# EUROPEAN PATENT SPECIFICATION

(11) **EP 3 536 274 B1**
(45) Date of publication and mention of the grant of the patent: **31.07.2024**
(21) Application number: 17867185.5
(22) Date of filing: 13.10.2017
(51) Int. Cl.: A61B 18/14, A61B 18/00, A61B 18/12

(54) **HIGH-FREQUENCY THERMAL THERAPY DEVICE**
HOCHFREQUENZ-WÄRMETHERAPIEVORRICHTUNG
DISPOSITIF DE THÉRAPIE THERMIQUE HAUTE FRÉQUENCE

(30) Priority: 04.11.2016 KR 20160146450
(43) Date of publication of application: 11.09.2019
(73) Proprietor: Starmed Co., Ltd., Gyeonggi-do 10442 (KR)
(72) Inventor: SHIN, Kyung Hoon, Gimpo-si Gyeonggi-do 10111 (KR); LIM, Hyuk, Paju-si Gyeonggi-do 10872 (KR)
(74) Representative: Zinkler, Franz
(86) International application number: PCT/KR2017/011305
(87) International publication number: WO 2018/084445

(56) References cited:
- EP-A1- 2 851 026
- KR-A- 20110 014 011
- KR-A- 20120 126 706
- KR-A- 20130 128 926
- KR-A- 20150 141 256
- US-A- 5 545 193
- US-A- 5 562 720
- US-A- 6 117 131
- US-A- 6 129 724
- US-A1- 2002 111 617
- US-A1- 2013 096 549
- US-A1- 2015 196 357
- US-A1- 2016 022 353
- US-B1- 6 356 790
- US-B2- 8 538 501

## Description

### TECHNICAL FIELD

The present disclosure relates to a high-frequency thermal therapy device, and more particularly, to a high-frequency thermal therapy device capable of cauterizing a target lesion with an electrode inserted into a body.

### BACKGROUND ART

Generally, if there is a cancerous tissue, etc. in the body organ (liver, thyroid, etc.), it is treated by a surgical method and a non-surgical method.

Recently, the non-surgical method is widely used as compared to the surgical method. As a non-surgical method, transarterial chemoembolization, percutaneous ethanol injection therapy, systemic anticancer chemotherapy, local thermal therapy, etc. are known, and among them, the local thermal therapy is the most effective method.

The local thermal therapy includes high-frequency thermal therapy, microwave cauterization, laser cauterization, etc., and among them, the high-frequency thermal therapy is the most effective, such that physicians or patients have been much demanding the above-described method.

Korean Patent Laid-Open Publication No. 10-2013-0128926 discloses a treatment device (hereinafter, referred to as a high-frequency thermal therapy device) used for the high-frequency thermal therapy.

Referring to Korean Patent Laid-Open Publication No. 10-2013-0128926, the conventional high-frequency thermal therapy device includes a radio frequency generator for generating a radio frequency current and an electrode for cauterizing a target lesion with the radio frequency current generated from the radio frequency generator.

The conventional high-frequency thermal therapy device according to such a conventional configuration has the electrode inserted into the body to radiate a radio frequency energy, thereby cauterizing and necrotizing the target lesion such as a cancer tissue, or heats the target lesion of the blood vessel such as varicose veins to damage the intravenous wall and to cause fibrosis of the blood vessel, thereby removing swollen blood vessel.

However, there has been a problem in that it is not possible to adjust the cauterization length of electrode in the conventional high-frequency thermal therapy device. Therefore, when the target lesion is smaller than the cauterization length of the electrode, a normal area around the target lesion has been damaged, and when the target lesion is larger than the cauterization length of the electrode, there has been a burden of cauterizing while moving the electrode. Meanwhile, considering such a problem, a plurality of high-frequency thermal therapy devices having the electrodes of different lengths are provided, and the high-frequency thermal therapy device having the electrode of the length suitable for the target lesion according to the target lesion is also used. However, in this case, there is a problem in that the procuring cost of the plurality of high-frequency thermal therapy devices increases, and the high-frequency thermal therapy device should be replaced according to the size of the target lesion.

US 8 538 501 B2 discloses a switching assembly including four input terminals and four output terminals respectively connected to the four input terminals by four switches. That is, US 8 538 501 B2 discloses that a plurality of output terminals are connected to a plurality of input terminals by a plurality of switches, but fails to disclose or suggest a bridge selectively connects the input terminal to at least one of the plurality of output terminals so as to change a cauterization length of a target lesion.

US 2015/196357 A1 discloses a multi-pole synchronous pulmonary artery radiofrequency ablation catheter.

US 2002/111617 A1 discloses an adjustable trans-urethral radio-frequency ablation.

US 6 129 724 A discloses systems and methods for forming elongated lesion patterns in body tissue using straight or curvilinear electrode elements.

US 5 545 193 A discloses a helically wound radio-frequency emitting electrodes for creating lesions in body tissue.

US 2013/096549 A1 discloses a method and apparatus for precisely controlling the size and shape of radio frequency ablations.

EP 2 851 026 A1 discloses an overlapping bipolar electrode for high-frequency heat treatment.

### DETAILED DESCRIPTION OF INVENTION

### TECHNICAL PROBLEM

Therefore, an object of the present disclosure is to provide a high-frequency thermal therapy device capable of adjusting the cauterization length of an electrode.

### TECHNICAL SOLUTION

For achieving this object, the present disclosure provides a high-frequency thermal therapy device including an electrode for cauterizing a target lesion; a radio frequency generator for generating a radio frequency current; and a switch for opening and closing an electric circuit extending from the radio frequency generator to the electrode, and a cauterization length of the electrode is changed according to an operation of the switch.

The electrode may include a body configured to extend in one direction; a first electrode disposed at one end portion of the body; and a second electrode disposed at another end portion of the body, and spaced apart from the first electrode. Each of the first electrode and the second electrode may be capable of cauterizing the target lesion.

The first electrode may include a first active electrode wound in a spiral shape along an outer circumferential surface of the body at one end portion of the body; and a first passive electrode wound in a spiral shape along the outer circumferential surface of the body at the one end portion of the body, and wound in parallel with the first active electrode. The first electrode may be configured to cauterize by the radio frequency radiation between the first active electrode and the first passive electrode.

The second electrode may include a second active electrode wound in a spiral shape along the outer circumferential surface of the body at another end portion of the body; and a second passive electrode wound in a spiral shape along the outer circumferential surface of the body at the another end portion of the body, and wound in parallel with the second active electrode. The second electrode may be configured to cauterize by the radio frequency radiation between the second active electrode and the second passive electrode.

According to the operation of the switch, the first active electrode and the first passive electrode may be electrically connected to the radio frequency generator, the second active electrode and the second passive electrode may also be electrically connected to the radio frequency generator, and the first active electrode, the first passive electrode, the second active electrode, and the second passive electrode may also be electrically connected to the radio frequency generator.

The switch may include a first terminal extending from an active terminal of the radio frequency generator; a second terminal extending from a passive terminal of the radio frequency generator, and constituting a pair of input terminals with the first terminal; a third terminal extending from the first active electrode; a fourth terminal extending from the first passive electrode, and constituting a pair of first output terminals with the third terminal; a fifth terminal extending from the second active electrode; a sixth terminal extending from the second passive electrode, and constituting a pair of second output terminals with the fifth terminal; and a bridge for connecting the pair of input terminals to or blocking the pair of input terminals from the pair of first output terminals and the pair of second output terminals.

The bridge may include a first bridge for connecting the input terminal with the first output terminal; a second bridge for connecting the input terminal with the second output terminal; and a third bridge for connecting the input terminal with the first output terminal and the second output terminal. Any one of the first bridge, the second bridge, and the third bridge may be activated to electrically connect the radio frequency generator to the electrode, or all of the first bridge, the second bridge, and the third bridge may be inactivated to be configured to electrically disconnect the radio frequency generator from the electrode.

The first bridge may include a first active bridge for connecting the first terminal with the third terminal; and a first passive bridge for connecting the second terminal with the fourth terminal. Then, the second bridge may include a second active bridge for connecting the first terminal with the fifth terminal; and a second passive bridge for connecting the second terminal with the sixth terminal. Then, the third bridge may include a third active bridge for connecting the first terminal with the third terminal and the fifth terminal; and a third passive bridge for connecting the second terminal with the fourth terminal and the sixth terminal.

The length of the first electrode may be configured differently from the length of the second electrode in the extending direction of the body.

Meanwhile, the electrode may include a plurality of electrode bodies capable of cauterizing the target lesion, each electrode body including an active electrode body and a passive electrode body facing each other to be configured such that a cauterization may be performed by the radio frequency radiation between the active electrode body and the passive electrode body, and may be configured such that at least one of the plurality of electrode bodies may be electrically connected to the radio frequency generator by the operation of the switch, and so that the cauterization length is changed according to the number of the electrode bodies electrically connected to the radio frequency generator among the plurality of electrode bodies.

Then, the active electrode body may be configured to have an annular shape, and the passive electrode body may be configured to have an annular shape parallel to the active electrode body.

### ADVANTAGEOUS EFFECTS

The high-frequency thermal therapy device according to the present disclosure provides the electrode having the first electrode and the second electrode capable of cauterizing the target lesion, respectively, and according to the operation of the switch, the power may be applied to the first electrode, the power may be applied to the second electrode, or the power may be applied to both the first electrode and the second electrode, thereby adjusting the cauterization length of the electrode. As a result, it is possible to prevent the normal area around the target lesion from being damaged, facilitate the treatment, and reduce the cost for procuring the equipment.

### BRIEF DESCRIPTION OF DRAWINGS

FIG. 1 is a front diagram illustrating a high-frequency thermal therapy device according to an embodiment of the present disclosure.
FIG. 2 is a perspective diagram illustrating an electrode and a handle of FIG. 1.
FIG. 3 is a circuit diagram illustrating a state where a first electrode of FIG. 1 has been connected with the radio frequency generator.
FIG. 4 is a circuit diagram illustrating a state where a second electrode of FIG. 1 has been connected with the radio frequency generator.
FIG. 5 is a circuit diagram illustrating a state where the first electrode and the second electrode of FIG. 1 have been connected with the radio frequency generator.
FIG. 6 is a circuit diagram illustrating a state where the first electrode and the second electrode of FIG. 1 have been disconnected with the radio frequency generator.

### BEST MODE

Hereinafter, a high-frequency thermal therapy device according to the present disclosure will be described in detail with reference to the accompanying drawings.

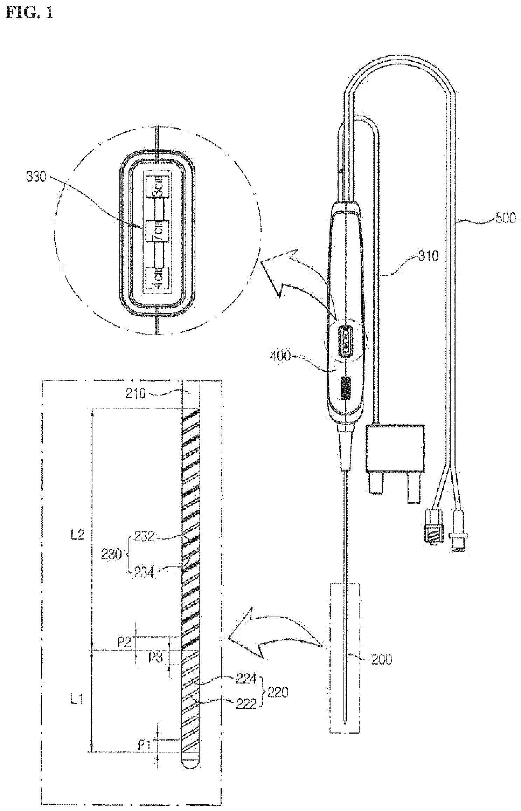

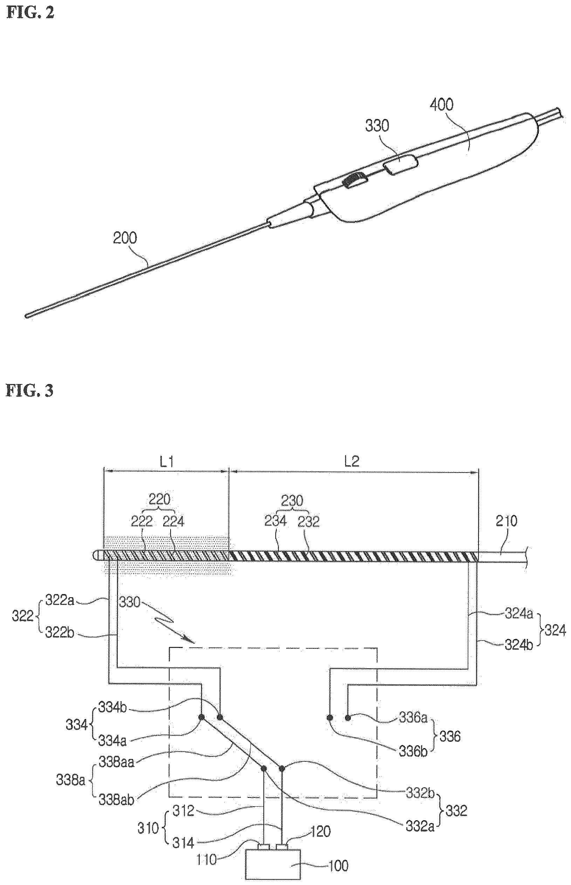

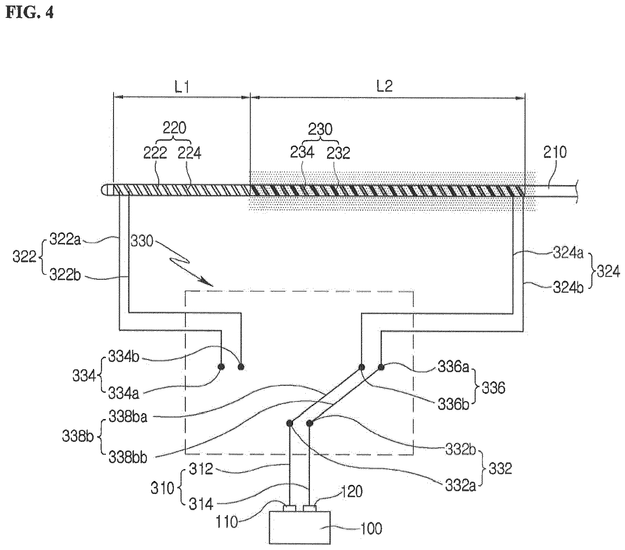

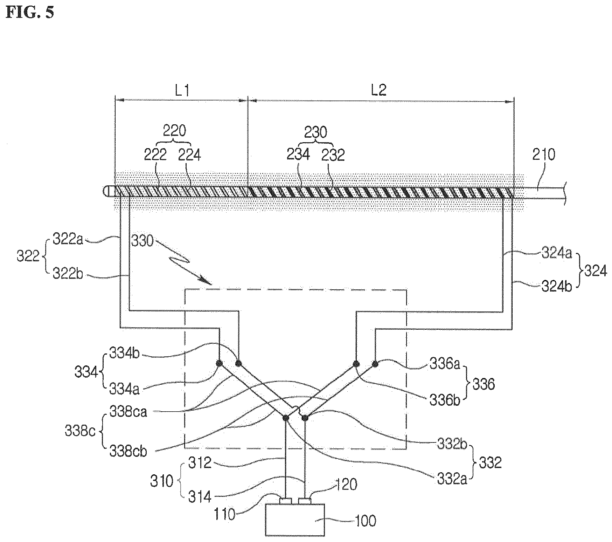

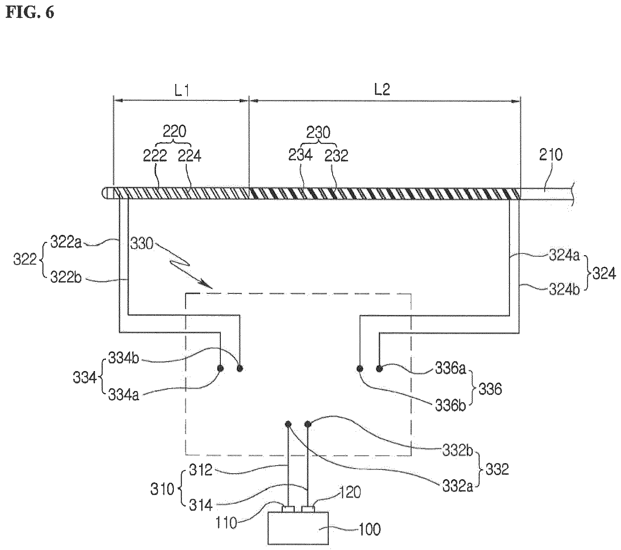

FIG. 1 is a front diagram illustrating a high-frequency thermal therapy device according to an embodiment of the present disclosure, FIG. 2 is a perspective diagram illustrating an electrode and a handle of FIG. 1, FIG. 3 is a circuit diagram illustrating a state where a first electrode of FIG. 1 has been connected with the radio frequency generator, FIG. 4 is a circuit diagram illustrating a state where a second electrode of FIG. 1 has been connected with the radio frequency generator, FIG. 5 is a circuit diagram illustrating a state where the first electrode and the second electrode of FIG. 1 have been connected with the radio frequency generator, and FIG. 6 is a circuit diagram illustrating a state where the first electrode and the second electrode of FIG. 1 have been disconnected with the radio frequency generator.

Referring to FIGS. 1 to 6, a high-frequency thermal therapy device according to an embodiment of the present disclosure may include a radio frequency generator 100 for generating a radio frequency current, an electrode 200 for cauterizing a target lesion with the radio frequency current generated from the radio frequency generator 100, and an electric circuit extending from the radio frequency generator 100 to the electrode 200. Herein, the high-frequency thermal therapy device may further include a cooling device 500 for cooling the electrode.

The radio frequency generator 100 may be configured such that a radio frequency AC is supplied to the electrode 200 through an active terminal 110 and a passive terminal 120.

The electrode 200 may be configured as a so-called oblique type bipolar electrode in which a heat generation range is configured to have a cylindrical shape, and may be configured such that heat may be generated from two portions thereof independently.

Specifically, the electrode 200 may include a body 210 configured to extend in one direction, a first electrode 220 disposed at one end portion of the body 210, and a second electrode 230 disposed at the other end portion of the body 210.

The body 210 may be configured to have a needle shape in order to be easily inserted into a target lesion tissue.

The first electrode 220 may include a first active electrode 222 that is wound in a spiral shape along the outer circumferential surface of the body 210 at one end portion of the body 210 and a first passive electrode 224 that is wound in a spiral shape along the outer circumferential surface of the body 210 at one end portion of the body 210 and wound in parallel with the first active electrode 222.

The first active electrode 222 and the first passive electrode 224 may be configured by winding in two or more times in parallel at the same lead angle with each other. That is, the first passive electrode 224 is wound in a spiral shape between the first active electrode 222 wound in a spiral shape so that the first active electrode 222 and the first passive electrode 224 are alternately wound, and the alternating interval between the first active electrode 222 and the first passive electrode 224 may be constantly formed (i.e., the pitch P1 between the first active electrode 222 and the first passive electrode 224 may be constantly formed).

Then, the first active electrode 222 and the first passive electrode 224 may be configured such that the first active electrode 222 is connected to the active terminal 110 of the radio frequency generator 100, and the first passive electrode 224 is connected to the passive terminal 120 of the radio frequency generator 100 through the electric circuit.

In the first electrode 220 according to such configuration, when the radio frequency current generated from the radio frequency generator 100 is applied to the first active electrode 222 and the first passive electrode 224 through the electric circuit, the target lesion may be cauterized by the radio frequency radiation generated between the first active electrode 222 and the first passive electrode 224.

At this time, the first electrode 220 may provide heat generated around the intermediate point of the pitch P1 between the first active electrode 222 and the first passive electrode 224 at the time of radiating the radio frequency energy between the first active electrode 222 and the first passive electrode 224. At this time, since the pitch P1 between the first active electrode 222 and the first passive electrode 224 is shorter than the diameter of the body 210, the heat generation range may be formed to have a tubular shape surrounding the body 210, and since the pitch P1 between the first active electrode 222 and the first passive electrode 224 is constant, the heat generation range may be formed to have a cylindrical shape having a rectangular vertical section.

The second electrode 230 may include a second active electrode 232 that is wound in a spiral shape along the outer circumferential surface of the body 210 at the other end portion of the body 210 and a second passive electrode 234 that is wound in a spiral shape along the outer circumferential surface of the body 210 at the other end portion of the body 210 and wound in parallel with the second active electrode 232.

The second active electrode 232 and the second passive electrode 234 may be configured by winding in two or more times in parallel at the same lead angle with each other. That is, the second passive electrode 234 is wound in a spiral shape between the second active electrode 232 wound in a spiral shape so that the second active electrode 232 and the second passive electrode 234 are alternately wound, and the alternating interval between the second active electrode 232 and the second passive electrode 234 may be constantly formed (i.e., the pitch P2 between the second active electrode 232 and the second passive electrode 234 may be constantly formed). Herein, the lead angles and the pitches of the first electrode 220 and the second electrode 230 may be preferably configured at the same level entirely so that the quality of cauterization by the first electrode 220 and the quality of cauterization by the second electrode 230 become the same level as each other. That is, it may be preferable that the lead angle of the first active electrode 222, the lead angle of the first passive electrode 224, the lead angle of the second active electrode 232, and the lead angle of the second passive electrode 234 are configured to be the same as each other, and the pitch P1 between the first active electrode 222 and the first passive electrode 224, the pitch P2 between the second active electrode 232 and the second passive electrode 234, and the pitch P3 between the first electrode 220 and the second electrode 230 are configured to be the same as each other.

Then, the second active electrode 232 and the second passive electrode 234 may have the second active electrode 232 connected to the active terminal 110 of the radio frequency generator 100, and have the second passive electrode 234 connected to the passive terminal 120 of the radio frequency generator 100 through the electric circuit.

In the second electrode 230 according to such configuration, when the radio frequency current generated from the radio frequency generator 100 is applied to the second active electrode 232 and the second passive electrode 234 through the electric circuit, the target lesion may be cauterized by the radio frequency radiation generated between the second active electrode 232 and the second passive electrode 234.

At this time, the second electrode 230 may provide heat generated around the intermediate point of the pitch P2 between the second active electrode 232 and the second passive electrode 234 at the time of radiating the radio frequency energy between the second active electrode 232 and the second passive electrode 234. At this time, since the pitch P2 between the second active electrode 232 and the second passive electrode 234 is shorter than the diameter of the body 210, the heat generation range may be configured to have a tubular shape surrounding the body 210, and since the pitch P2 between the second active electrode 232 and the second passive electrode 234 are constant, the heat generation range may have a cylindrical shape having a rectangular vertical section.

Herein, for cauterizing by the first electrode 220 and the second electrode 230 separately from each other, the second active electrode 232 and the second passive electrode 234 may be configured to be spaced apart from the first active electrode 222 and the first passive electrode 224 in the extending direction of the body 210 not to be contacted with the first active electrode 222 and the first passive electrode 224. That is, the first electrode 220 and the second electrode 230 may be configured such that the current received into the first active electrode 222 and the first passive electrode 224 does not flow into the second active electrode 232 and the second passive electrode 234, and the vice versa.

Meanwhile, in the first electrode 220 and the second electrode 230, the length L1 of the first electrode 220 may be configured differently from the length L2 of the second electrode 230 so that the cauterization length (i.e., the length in the extending direction of the body 210) by the first electrode 220 is different from the cauterization length (i.e., the length in the extending direction of the body 210) by the second electrode 230. In the present embodiment, the length L1 of the first electrode 220 may be configured at a level of about 3cm, and the length L2 of the second electrode 230 may be configured at a level of about 4cm.

The electric circuit may include a lead-in wire 310 extending from the radio frequency generator 100, a lead-out wire 320 extending from the electrode 200, and a switch 330 for connecting and disconnecting the lead-in wire 310 and the lead-out wire 320.

Herein, the switch 330 may be disposed at a handle 400 provided at one side of the electrode 200 (more accurately, the body 210), the lead-in wire 310 may be extended into the handle 400 from the radio frequency generator 100 to be connected with the switch 330, and the lead-out wire 320 may be extended from the switch 330 to be connected to the first electrode 220 and the second electrode 230 through the handle 400 and the body 210.

The lead-in wire 310 may include an active lead-in wire 312 extending from the active terminal 110 of the radio frequency generator 100 and a passive lead-in wire 314 extending from the passive terminal 120 of the radio frequency generator 100.

The lead-out wire 320 may include a first active lead-out wire 322a extending from the first active electrode 222; a first passive lead-out wire 322b extending from the first passive electrode 224, and constituting a pair of first lead-out wires 322 with the first active lead-out wire 322a; a second active lead-out wire 324a extending from the second active electrode 232; and a second passive lead-out wire 324b extending from the second passive electrode 234, and constituting a pair of second lead-out wires 324 with the second active lead-out wire 324a.

The switch 330 may include a first terminal 332a connected with the active lead-in wire 312; a second terminal 332b connected with the passive lead-in wire 314, and constituting a pair of input terminals 332 with the first terminal 332a; a third terminal 334a connected with the first active lead-out wire 322a; a fourth terminal 334b connected with the first passive lead-out wire 322b, and constituting a pair of first output terminals 334 with the third terminal 334a; a fifth terminal 336b connected with the second active lead-out wire 324a; a sixth terminal 336a connected with the second passive lead-out wire 324b, and constituting a pair of second output terminals 336 with the fifth terminal 336b; and a bridge 338 for connecting and blocking the pair of input terminals 332 to the pair of first output terminals 334 and the pair of second output terminals 336.

The bridge 338 may include a first bridge 338a for connecting the input terminal 332 with the first output terminal 334, a second bridge 338b for connecting the input terminal 332 with the second output terminal 336, and a third bridge 338c for connecting the input terminal 332 with the first output terminal 334 and the second output terminal 336.

The first bridge 338a may include a first active bridge 338aa for connecting the first terminal 332a with the third terminal 334a and a first passive bridge 338ab for connecting the second terminal 332b with the fourth terminal 334b. Herein, the first active bridge 338aa and the first passive bridge 338ab may be configured to be interlocked with each other. That is, the first active bridge 338aa and the first passive bridge 338ab may be configured such that when the first active bridge 338aa is activated (i.e., the first terminal 332a and the third terminal 334a are connected), the first passive bridge 338ab is also activated (i.e., the second terminal 332b and the fourth terminal 334b are connected), and when the first active bridge 338aa is inactivated (i.e., the first terminal 332a and the third terminal 334a are disconnected), the first passive bridge 338ab is also inactivated (i.e., the second terminal 332b and the fourth terminal 334b are disconnected).

The second bridge 338b may include a second active bridge 338ba for connecting the first terminal 332a with the fifth terminal 336b and a second passive bridge 338bb for connecting the second terminal 332b with the sixth terminal 336a. Herein, the second active bridge 338ba and the second passive bridge 338bb may be configured to be interlocked with each other. That is, the second active bridge 338ba and the second passive bridge 338bb may be configured such that when the second active bridge 338ba is activated (i.e., the first terminal 332a and the fifth terminal 336b are connected), the second passive bridge 338bb is also activated (i.e., the second terminal 332b and the sixth terminal 336a are connected), and when the second active bridge 338ba is inactivated (i.e., the first terminal 332a and the fifth terminal 336b are disconnected), the second passive bridge 338bb is also inactivated (i.e., the second terminal 332b and the sixth terminal 336a are disconnected).

The third bridge 338c may include a third active bridge 338ca for connecting the first terminal 332a with the third terminal 334a and the fifth terminal 336b and a third passive bridge 338cb for connecting the second terminal 332b with the fourth terminal 334b and the sixth terminal 336a. Herein, the third active bridge 338ca may be branched to simultaneously connect the first terminal 332a with the third terminal 334a and the fifth terminal 336b, and the third passive bridge 338cb may be branched to simultaneously connect the second terminal 332b with the fourth terminal 334b and the sixth terminal 336a. Then, the third active bridge 338ca and the third passive bridge 338cb may be configured to be interlocked with each other. That is, the third active bridge 338ca and the third passive bridge 338cb may be configured such that when the third active bridge 338ca is activated (i.e., the first terminal 332a is connected with the third terminal 334a and the fifth terminal 336b), the third passive bridge 338cb is also activated (i.e., the second terminal 332b is connected with the fourth terminal 334b and the sixth terminal 336a), and when the third active bridge 338ca is inactivated (i.e., the first terminal 332a is disconnected with the third terminal 334a and the fifth terminal 336b), the third passive bridge 338cb is also inactivated (i.e., the second terminal 332b is disconnected with the fourth terminal 334b and the sixth terminal 336a).

In the electric circuit according to such configuration, any one of the first bridge 338a, the second bridge 338b, and the third bridge 338c may be activated to supply the current generated from the radio frequency generator 100 to at least one of the first electrode 220 and the second electrode 230, or all of the first bridge 338a, the second bridge 338b, and the third bridge 338c may be inactivated to electrically disconnect the first electrode 220 and the second electrode 230 by the radio frequency generator 100.

Hereinafter, the operation and effect of the high-frequency thermal therapy device according to the present embodiment will be described.

That is, as illustrated in FIG. 6, in the high-frequency thermal therapy device according to the present embodiment, the electrode 200 may be inserted into the body of a patient in a state where all of the first bridge 338a, the second bridge 338b, and the third bridge 338c of the switch 330 are inactivated (an OFF state) so that the first electrode 220 or the second electrode 230 of the electrode 200 is disposed at the target lesion, and one of the first bridge 338a, the second bridge 338b, and the third bridge 338c of the switch 330 may be activated to cauterize the target lesion, and the cauterization length thereof may be changed.

More specifically, when the length of the target lesion to be cauterized is a level of the length L1 of the first electrode 220, the first electrode 220 may be disposed at the target lesion, and as illustrated in FIG. 3, when the first bridge 338a is activated, the target lesion may be cauterized at the length of approximately the same level as the length L1 of the first electrode 220 while the radio frequency radiation is generated between the first active electrode 222 to which a current is applied from the active terminal 110 of the radio frequency generator 100 through the active lead-in wire 312, the first terminal 332a, the first active bridge 338aa, the third terminal 334a, and the first active lead-out wire 322a, and the first passive electrode 224 to which a current is applied from the passive terminal 120 of the radio frequency generator 100 through the passive lead-in wire 314, the second terminal 332b, the first passive bridge 338ab, the fourth terminal 334b, and the first passive lead-out wire 322b. At this time, the cauterization cannot occur in the second electrode 230.

Meanwhile, when the length of the target lesion to be cauterized is a level of the length L2 of the second electrode 230, the second electrode 230 may be disposed at the target lesion, and as illustrated in FIG. 4, when the second bridge 338b is activated, the target lesion may be cauterized at the length of approximately the same level as the length L2 of the second electrode 230 while the radio frequency radiation is generated between the second active electrode 232 to which a current is applied from the active terminal 110 of the radio frequency generator 100 through the active lead-in wire 312, the first terminal 332a, the second active bridge 338ba, the fifth terminal 336b, and the second active lead-out wire 324a, and the second passive electrode 234 to which a current is applied from the passive terminal 120 of the radio frequency generator 100 through the passive lead-in wire 314, the second terminal 332b, the second passive bridge 338bb, the sixth terminal 336a, and the second passive lead-out wire 324b. At this time, the cauterization cannot occur in the first electrode 220.

Meanwhile, when the length of the target lesion to be cauterized is a level of the sum of the length L1 of the first electrode 220 and the length L2 of the second electrode 230, the first electrode 220 and the second electrode 230 may be disposed at the target lesion, and as illustrated in FIG. 5, when the third bridge 338c is activated, heat is generated from both the first electrode 220 and the second electrode 230 so that the target lesion may be cauterized at the length of approximately the same level as the sum of the length L1 of the first electrode 220 and the length L2 of the second electrode 230. That is, a current applied to the active lean-in wire 312 and the first terminal 332a from the active terminal 110 of the radio frequency generator 100 may be branched to the third terminal 334a and the fifth terminal 336b by the third active bridge 338ca, a current branched to the third terminal 334a may be applied to the first active electrode 222 through the first active lead-out wire 322a, and a current branched to the fifth terminal 336b may be applied to the second active electrode 232 through the second active lead-out wire 324a. Then, a current applied to the passive lead-in wire 314 and the second terminal 332b from the passive terminal 120 of the radio frequency generator 100 may be branched to the fourth terminal 334b and the sixth terminal 336a by the third passive bridge 338cb, a current branched to the fourth terminal 334b may be applied to the first passive electrode 224 through the first passive lead-out wire 322b, and a current branched to the sixth terminal 336a may be applied to the second passive electrode 234 through the second passive lead-out wire 324b. Then, one side of the target lesion may be cauterized at the length of the same level as the length L1 of the first electrode 220 while the radio frequency radiation is generated between the first active electrode 222 and the first passive electrode 224. At the same time, the other side of the target lesion may be cauterized at the length of the same level as the length L2 of the second electrode 230 while the radio frequency radiation is generated between the second active electrode 232 and the second passive electrode 234. Therefore, the entire cauterization length of the target lesion may be the same level as the sum of the length L1 of the first electrode 220 and the length L2 of the second electrode 230.

After the cauterization has been completed, as illustrated in FIG. 6, the switch 330 may be inactivated (i.e., all of the first bridge 338a, the second bridge 338b, and the third bridge 338c are inactivated), and then the electrode 200 may be drawn out of the patient's body, thereby completing the treatment.

Herein, the high-frequency thermal therapy device of the present embodiment provides the first electrode 220 and the second electrode 230 capable of cauterizing the target lesion, respectively, on the electrode 200, and according to an operation of the switch 330, a power may be applied to the first electrode 220, a power may be applied to the second electrode 230, or a power may be applied to both the first electrode 220 and the second electrode 230, thereby adjusting the cauterization length of the electrode 200.

Therefore, it is possible to prevent the normal area around the target lesion from being damaged, facilitate the treatment, and reduce the procuring cost of the equipment.

Meanwhile, in the present embodiment, the lead angle of the first electrode 220 may be configured to be the same level as the lead angle of the second electrode 230, and the pitch P1 of the first electrode 220 may be configured to be the same level as the pitch P2 of the second electrode 230 so that the cauterization radius by the first electrode 220 and the cauterization radius by the second electrode 230 become the same level. However, the lead angle of the first electrode 220 may be configured to be a level differently from the lead angle of the second electrode 230, and the pitch P1 of the first electrode 220 may also be configured to be a level differently from the pitch P2 of the second electrode 230 so that the cauterization radius by the first electrode 220 and the cauterization radius by the second electrode 230 become a level differently from each other.

Meanwhile, in the present embodiment, the distance between the first electrode 220 and the second electrode 230 may be configured at the same level as the pitch P1 of the first electrode 220 and the pitch P2 of the second electrode 230 so that the cauterization area by the first electrode 220 and the cauterization area of the second electrode 230 may be connected to each other, and the first electrode 220 and the second electrode 230 may be configured such that the opposite polarities are opposite to each other (e.g., the second passive electrode 234 is configured opposite to the first active electrode 222, or the second active electrode 232 is configured opposite to the first passive electrode 224 at the area where the first electrode 220 and the second electrode 230 are adjacent to each other). However, in order to suppress discharge from occurring between the first electrode 220 and the second electrode 230, the distance between the first electrode 220 and the second electrode 230 may be configured longer than the pitch P1 of the first electrode 220 and the pitch P2 of the second electrode 230, and the first electrode 220 and the second electrode 230 may also be configured such that the same polarities are opposite to each other (e.g., the second active electrode 232 is configured opposite to the first active electrode 222, or the second passive electrode 234 is configured opposite to the first passive electrode 224 at the area where the first electrode 220 and the second electrode 230 are adjacent to each other).

Meanwhile, in the present embodiment, the switch 330 provides all of the first bridge 338a, the second bridge 338b, and the third bridge 338c, and any one of the first bridge 338a, the second bridge 338b, and the third bridge 338c may be activated, or may be all inactivated according to a button operation of the switch 330. However, the present embodiment is not limited thereto, and various embodiments for the switch 330 may be present within the range in which when the cauterization is performed, a current is applied to at least one of the first electrode 220 and the second electrode 230, and when the cauterization is not performed, a current is blocked to both the first electrode 220 and the second electrode 230.

Meanwhile, in the present disclosure the electrode 200 is configured as an oblique type bipolar electrode, but although not illustrated separately, it may be configured as an annular bipolar electrode. That is, the electrode may include a plurality of electrode bodies, each electrode may include an active electrode body and a passive electrode body parallel to each other, and the active electrode body and the passive electrode body may be configured to have an annular shape, respectively. Then, the plurality of electrode bodies may be applied with a power through an electric circuit and a switch, and the power may be applied to at least one of the plurality of electrode bodies according to the operation of the switch, thereby adjusting the cauterization length.

### INDUSTRIAL APPLICABILITY

The present disclosure provides the high-frequency thermal therapy device capable of adjusting the cauterization length of the electrode.

## Claims

1. A high-frequency thermal therapy device, comprising:
a handle (400);
an elongated body (210) extending from the handle (400);
a first electrode (220) disposed at one portion of the body (210) for cauterizing a target lesion and including a first active electrode (222) and a first passive electrode (224) wound in a spiral shape in parallel with each other along an outer circumferential surface of the body (210);
a second electrode (230) disposed at another portion of the body (210) for cauterizing the target lesion and including a second active electrode (232) and a second passive electrode (234) wound in a spiral shape in parallel with each other along the outer circumferential surface of the body (210);
a radio frequency generator (100) for generating a radio frequency current;
an electric circuit for connecting the radio frequency generator (100) to the first (220) and second (230) electrodes; and
a switch (330) comprising a first bridge (338a), a second bridge (338b) and a third bridge (338c) disposed on the handle (400) for opening or closing the electric circuit,
wherein a length of the first electrode (220) is different from a length of the second electrode (230) in the extending direction of the body (210),
wherein the switch (330) includes:
a pair of input terminals (332) connected to the radio frequency generator (100);
a pair of first output terminals (334) respectively connected to the first active electrode (222) and the first passive electrode (224);
a pair of second output terminals (336) respectively connected to the second active electrode (232) and the second passive electrode (234);
the bridges (338a, 338b, 338c) are configured to selectively connect the pair of input terminals (332) to the pair of first output terminals (334), to the pair of second output terminals (336), or to both, using the first bridge (338a), the second bridge (338b) and the third bridge (338c) respectively so as to change a cauterization length of the target lesion;
wherein each bridge (338a, 338b, 338c) includes an active bridge and a passive bridge, which are configured to be interlocked with each other such that when their active bridge is activated to connect one of the pair of input terminals (332) to one of the pair of the first output terminals (334) and/or to one of the pair of the second output terminals (336), the passive bridge is also activated to connect the other of the pair of input terminals (332) to the other of the pair of the first output terminals (334) and/or to the other of the pair of the second output terminals (336), and when the active bridge is inactivated to disconnect one of the pair of input terminals (332) to one of the pair of the first output terminals (334) and/or to one of the pair of the second output terminals (336), the passive bridge is also inactivated to disconnect the other of the pair of input terminals (332) to the other of the pair of the first output terminals (334) and/or to the other of the pair of the second output terminals (336).

2. The high-frequency thermal therapy device of claim 1,
wherein according to the operation of the switch (330),
the first active electrode (222) and the first passive electrode (224) are electrically connected to the radio frequency generator (100);
the second active electrode (232) and the second passive electrode (234) are electrically connected to the radio frequency generator (100); or
the first active electrode (222), the first passive electrode (224), the second active electrode (232), and the second passive electrode (234) are electrically connected to the radio frequency generator (100).

3. The high-frequency thermal therapy device of claim 2,
wherein the switch (330) comprises:
a first terminal (332a) extending from an active terminal (110) of the radio frequency generator (100);
a second terminal (332b) extending from a passive terminal (120) of the radio frequency generator (100), and constituting the pair of input terminals (332) with the first terminal (332a);
a third terminal (334a) extending from the first active electrode (222);
a fourth terminal (334b) extending from the first passive electrode (224), and constituting the pair of first output terminals (334) with the third terminal (334a);
a fifth terminal (336b) extending from the second active electrode (232);
a sixth terminal (336a) extending from the second passive electrode (234), and constituting the pair of second output terminals (336) with the fifth terminal (336b); and
wherein the bridge (338) is configured to connect the pair of input terminals (332) to or block the pair of input terminals (332) from the pair of first output terminals (334) and the pair of second output terminals (336).

4. The high-frequency thermal therapy device of claim 4, wherein the first bridge (338a) comprises:
a first active bridge (338aa) for connecting the first terminal (332a) with the third terminal (334a); and
a first passive bridge (338ab) for connecting the second terminal (332b) with the fourth terminal (334b),
wherein the second bridge (338b) comprises:
a second active bridge (338ba) for connecting the first terminal (332a) with the fifth terminal (336b); and
a second passive bridge (338bb) for connecting the second terminal (332b) with the sixth terminal (336a), and
wherein the third bridge (338c) comprises:
a third active bridge (338ca) for connecting the first terminal (332a) with the third terminal (334a) and the fifth terminal (336b); and
a third passive bridge (338cb) for connecting the second terminal (332b) with the fourth terminal (334b) and the sixth terminal (336a).

## Patentansprüche

1. Eine Hochfrequenzthermotherapievorrichtung, die folgende Merkmale aufweist:
einen Griff (400);
einen länglichen Körper (210), der sich von dem Griff (400) erstreckt;
eine erste Elektrode (220), die an einem Abschnitt des Körpers (210) zum Kauterisieren einer Zielläsion angeordnet ist, und die eine erste aktive Elektrode (222) und eine erste passive Elektrode (224) umfasst, die entlang einer Außenumfangsoberfläche des Körpers (210) in einer Spiralform parallel zueinander gewickelt sind;
eine zweite Elektrode (230), die an einem anderen Abschnitt des Körpers (210) zum Kauterisieren der Zielläsion angeordnet ist, und die eine zweite aktive Elektrode (232) und eine zweite passive Elektrode (234) umfasst, die entlang der Außenumfangsoberfläche des Körpers (210) in einer Spiralform parallel zueinander gewickelt sind;
einen Hochfrequenzgenerator (100) zum Erzeugen eines Hochfrequenzstroms;
eine elektrische Schaltung zum Verbinden des Hochfrequenzgenerators (100) mit der ersten (220) und zweiten (230) Elektrode; und
einen Schalter (330), der eine erste Brücke (338a), eine zweite Brücke (338b) und eine dritte Brücke (338c) aufweist, die an dem Griff (400) angeordnet sind, zum Offnen oder Schließen der elektrischen Schaltung,
wobei eine Länge der ersten Elektrode (220) sich von einer Länge der zweiten Elektrode (230) in der Erstreckungsrichtung des Körpers (210) unterscheidet,
wobei der Schalter (330) folgende Merkmale umfasst:
ein Paar von Eingangsanschlüssen (332), die mit dem Hochfrequenzgenerator (100) verbunden sind;
ein Paar von ersten Ausgangsanschlüssen (334), die jeweils mit der ersten aktiven Elektrode (222) und der ersten passiven Elektrode (224) verbunden sind;
ein Paar von zweiten Ausgangsanschlüssen (336), die jeweils mit der zweiten aktiven Elektrode (232) und der zweiten passiven Elektrode (234) verbunden sind;
wobei die Brücken (338a, 338b, 338c) dazu konfiguriert sind, unter Verwendung der ersten Brücke (338a), der zweiten Brücke (338b) beziehungsweise der dritten Brücke (338c) das Paar von Eingangsanschlüssen (332) selektiv mit dem Paar von ersten Ausgangsanschlüssen (334), mit dem Paar von zweiten Ausgangsanschlüssen (336) oder mit beiden zu verbinden, um eine Kauterisierungslänge der Zielläsion zu ändern,
wobei jede Brücke (338a, 338b, 338c) eine aktive Brücke und eine passive Brücke umfasst, die dazu konfiguriert sind, ineinanderzugreifen, so dass, wenn die aktive Brücke derselben aktiviert ist, um einen des Paars von Eingangsanschlüssen (332) mit einem des Paars der ersten Ausgangsanschlüsse (334) und/oder mit einem des Paars der zweiten Ausgangsanschlüsse (336) zu verbinden, die passive Brücke ebenfalls aktiviert ist, um den anderen des Paars von Eingangsanschlüssen (332) mit dem anderen des Paars der ersten Ausgangsanschlüsse (334) und/oder dem anderen des Paars der zweiten Ausgangsanschlüsse (336) zu verbinden, und wenn die aktive Brücke inaktiviert ist, um einen des Paars von Eingangsanschlüssen (332) von einem des Paars der ersten Ausgangsanschlüsse (334) und/oder einem des Paars der zweiten Ausgangsanschlüsse (336) zu trennen, die passive Brücke ebenfalls inaktiviert ist, um den anderen des Paars von Eingangsanschlüssen (332) von dem anderen des Paars der ersten Ausgangsanschlüsse (334) und/oder dem anderen des Paars der zweiten Ausgangsanschlüsse (336) zu trennen.

2. Die Hochfrequenzthermotherapievorrichtung gemäß Anspruch 1,
bei der gemäß dem Betrieb des Schalters (330)
die erste aktive Elektrode (222) und die erste passive Elektrode (224) mit dem Hochfrequenzgenerator (100) elektrisch verbunden sind;
die zweite aktive Elektrode (232) und die zweite passive Elektrode (234) mit dem Hochfrequenzgenerator (100) elektrisch verbunden sind; oder
die erste aktive Elektrode (222), die erste passive Elektrode (224), die zweite aktive Elektrode (232) und die zweite passive Elektrode (234) mit dem Hochfrequenzgenerator (100) elektrisch verbunden sind.

3. Die Hochfrequenzthermotherapievorrichtung gemäß Anspruch 2,
bei der der Schalter (330) folgende Merkmale aufweist:
einen ersten Anschluss (332a), der sich von einem aktiven Anschluss (110) des Hochfrequenzgenerators (100) erstreckt;
einen zweiten Anschluss (332b), der sich von einem passiven Anschluss (120) des Hochfrequenzgenerators (100) erstreckt und mit dem ersten Anschluss (332a) das Paar von Eingangsanschlüssen (332) bildet;
einen dritten Anschluss (334a), der sich von der ersten aktiven Elektrode (222) erstreckt;
einen vierten Anschluss (334b), der sich von der ersten passiven Elektrode (224) erstreckt und mit dem dritten Anschluss (334a) das Paar von ersten Ausgangsanschlüssen (334) bildet;
einen fünften Anschluss (336b), der sich von der zweiten aktiven Elektrode (232) erstreckt;
einen sechsten Anschluss (336a), der sich von der zweiten passiven Elektrode (234) erstreckt und mit dem fünften Anschluss (336b) das Paar von zweiten Ausgangsanschlüssen (336) bildet; und
wobei die Brücke (338) dazu konfiguriert ist, das Paar von Eingangsanschlüssen (332) mit dem Paar von ersten Ausgangsanschlüssen (334) und dem Paar von zweiten Ausgangsanschlüssen (336) zu verbinden oder das Paar von Eingangsanschlüssen (332) von denselben zu trennen.

4. Die Hochfrequenzthermotherapievorrichtung gemäß Anspruch 4,
bei der die erste Brücke (338a) folgende Merkmale aufweist:
eine erste aktive Brücke (338aa) zum Verbinden des ersten Anschlusses (332a) mit dem dritten Anschluss (334a); und
eine erste passive Brücke (338ab) zum Verbinden des zweiten Anschlusses (332b) mit dem vierten Anschluss (334b),
wobei die zweite Brücke (338b) folgende Merkmale aufweist:
eine zweite aktive Brücke (338ba) zum Verbinden des ersten Anschlusses (332a) mit dem fünften Anschluss (336b); und
eine zweite passive Brücke (338bb) zum Verbinden des zweiten Anschlusses (332b) mit dem sechsten Anschluss (336a) und
wobei die dritte Brücke (338c) folgende Merkmale aufweist:
eine dritte aktive Brücke (338ca) zum Verbinden des ersten Anschlusses (332a) mit dem dritten Anschluss (334a) und dem fünften Anschluss (336b); und
eine dritte passive Brücke (338cb) zum Verbinden des zweiten Anschlusses (332b) mit dem vierten Anschluss (334b) und dem sechsten Anschluss (336a).

## Revendications

1. Appareil de thermothérapie à hautes fréquences, comprenant:
une poignée (400);
un corps allongé (210) s'étendant à partir de la poignée (400);
une première électrode (220) disposée au niveau d'une partie du corps (210) pour cautériser une lésion cible et comportant une première électrode active (222) et une première électrode passive (224) enroulées en forme de spirale de manière parallèle l'une à l'autre le long d'une surface circonférentielle extérieure du corps (210);
une deuxième électrode (230) disposée au niveau d'une autre partie du corps (210) pour cautériser la lésion cible et comportant une deuxième électrode active (232) et une deuxième électrode passive (234) enroulées en forme de spirale de manière parallèle l'une à l'autre le long de la surface circonférentielle extérieure du corps (210);
un générateur de radiofréquence (100) destiné à générer un courant de radiofréquence;
un circuit électrique destiné à connecter le générateur de radiofréquence (100) aux première (220) et deuxième (230) électrodes; et
un commutateur (330) comprenant un premier pont (338a), un deuxième pont (338b) et un troisième pont (338c) disposés sur la poignée (400) pour ouvrir ou fermer le circuit électrique,
dans lequel une longueur de la première électrode (220) est différente d'une longueur de la deuxième électrode (230) dans la direction d'extension du corps (210),
dans lequel le commutateur (330) comporte:
une paire de bornes d'entrée (332) connectées au générateur de radiofréquence (100);
une paire de premières bornes de sortie (334) connectées respectivement à la première électrode active (222) et à la première électrode passive (224);
une paire de deuxièmes bornes de sortie (336) connectées respectivement à 1a deuxième électrode active (232) et à la deuxième électrode passive (234);
les ponts (338a, 338b, 338c) sont configurés pour connecter sélectivement la paire de bornes d'entrée (332) à la paire de premières bornes de sortie (334), à la paire de deuxièmes bornes de sortie (336), ou aux deux, à l'aide respectivement du premier pont (338a), du deuxième pont (338b) et du troisième pont (338c), de manière à modifier une longueur de cautérisation de la lésion cible;
dans lequel chaque pont (338a, 338b, 338c) comporte un pont actif et un pont passif qui sont configurés pour être inter-verrouillés l'un avec l'autre de sorte que, lorsque leur pont actif est activé pour connecter l'une de la paire de bornes d'entrée (332) à l'une de la paire de premières bornes de sortie (334) et/ou à l'une de la paire de deuxièmes bornes de sortie (336), le pont passif est également activé pour connecter l'autre de la paire de bornes d'entrée (332) à l'autre de la paire de premières bornes de sortie (334) et/ou à l'autre de la paire de deuxièmes bornes de sortie (336) et, lorsque le pont actif est désactivé pour déconnecter l'une de la paire de bornes d'entrée (332) de l'une de la paire de premières bornes de sortie (334) et/ou de l'une de la paire de deuxièmes bornes de sortie (336), le pont passif est également désactivé pour déconnecter l'autre de la paire de bornes d'entrée (332) de l'autre de la paire de premières bornes de sortie (334) et/ou de l'autre de la paire de deuxièmes bornes de sortie (336).

2. Appareil de thermothérapie à hautes fréquences selon la revendication 1,
dans lequel, selon le fonctionnement du commutateur (330),
la première électrode active (222) et la première électrode passive (224) sont connectées électriquement au générateur de radiofréquence (100);
la deuxième électrode active (232) et la deuxième électrode passive (234) sont connectées électriquement au générateur de radiofréquence (100); ou
la première électrode active (222), la première électrode passive (224), la deuxième électrode active (232) et la deuxième électrode passive (234) sont connectées électriquement au générateur de radiofréquence (100).

3. Dispositif de thermothérapie à hautes fréquences selon la revendication 2,
dans lequel le commutateur (330) comprend:
une première borne (332a) s'étendant à partir d'une borne active (110) du générateur de radiofréquence (100);
une deuxième borne (332b) s'étendant à partir d'une borne passive (120) du générateur de radiofréquence (100), et constituant la paire de bornes d'entrée (332) avec la première borne (332a);
une troisième borne (334a) s'étendant à partir de la première électrode active (222);
une quatrième borne (334b) s'étendant à partir de la première électrode passive (224) et constituant la paire de premières bornes de sortie (334) avec la troisième borne (334a);
une cinquième borne (336b) s'étendant à partir de la deuxième électrode active (232);
une sixième borne (336a) s'étendant à partir de la deuxième électrode passive (234) et constituant la paire de deuxièmes bornes de sortie (336) avec la cinquième borne (336b); et
dans lequel le pont (338) est configuré pour connecter la paire de bornes d'entrée (332) à ou pour bloquer la paire de bornes d'entrée (332) de la paire de premières bornes de sortie (334) et de la paire de deuxièmes bornes de sortie (336).

4. Dispositif de thermothérapie à hautes fréquences selon la revendication 4,
dans lequel le premier pont (338a) comprend:
un premier pont actif (338aa) destiné à connecter la première borne (332a) au troisième borne (334a); et
un premier pont passif (338ab) destiné à connecter la deuxième borne (332b) au quatrième borne (334b),
dans lequel le deuxième pont (338b) comprend:
un deuxième pont actif (338ba) destiné à connecter la première borne (332a) à la cinquième borne (336b); et
un deuxième pont passif (338bb) destiné à connecter la deuxième borne (332b) à la sixième borne (336a), et
dans lequel le troisième pont (338c) comprend:
un troisième pont actif (338ca) destiné à connecter la première borne (332a) à la troisième borne (334a) et à la cinquième borne (336b); et
un troisième pont passif (338cb) destiné à connecter la deuxième borne (332b) à la quatrième borne (334b) et à la sixième borne (336a).
